# EUROPEAN PATENT APPLICATION

(11) **EP 3 195 942 A1**
(43) Date of publication of application: **26.07.2017**
(21) Application number: 16184059.0
(22) Date of filing: 12.08.2016
(51) Int. Cl.: B06B 1/06, G01H 11/08, A61B 8/00

(54) **ULTRASONIC PROBE AND METHOD OF MANUFACTURING THE SAME**

(30) Priority: 19.01.2016 KR 20160006460
(71) Applicant: Samsung Medison Co., Ltd., Hongcheon-gun, Gangwon-do, 25108 (KR)
(72) Inventor: Kim, Young-il, Gyeonggi-do (KR); Choi, Min-seog, Seoul (KR); Song, Jong-keun, Gyeonggi-do (KR)
(74) Representative: Land, Addick Adrianus Gosling

(57) **Abstract**

Provided are an ultrasonic probe (110) and a method of manufacturing the same. The ultrasonic probe (110) includes: a first unit (310) configured to generate an ultrasonic wave from a first electrical signal or generate a second electrical signal from an echo signal of the ultrasonic wave; a second unit (320) configured to provide the first electrical signal to the first unit (310) or receive the second electrical signal from the first unit (310); and a third unit (330) configured to electrically connect the first unit (310) to the second unit (320), the third unit (330) comprising a plurality of conductive bumps (450) spaced apart from one another and a non-conductive paste or film (460) that surrounds the plurality of conductive bumps (450).

## Description

This application claims the benefit of Korean Patent Application No. 10-2016-0006460, filed on January 19, 2016, in the Korean Intellectual Property Office, the disclosure of which is incorporated herein in its entirety by reference.

The present disclosure relates to an ultrasonic probe with improved yield and a method of manufacturing the same.

An ultrasonic diagnostic apparatus irradiates an ultrasonic wave to a subject, such as a human or an animal, detects an echo signal reflected from the subject, displays a tomographic image of a tissue on a monitor, and provides information for diagnosis of the subject. The ultrasonic diagnostic apparatus includes an ultrasonic probe so as to transmit the ultrasonic wave to the subject and receive the echo signal from the subject.

The ultrasonic probe includes a piezoelectric layer which performs a conversion between an ultrasonic wave and an electrical signal. The piezoelectric layer includes an array of a plurality of piezoelectric elements. Therefore, the ultrasonic diagnostic apparatus having the above-described configuration irradiates an ultrasonic wave to a subject, converts an echo signal reflected from the subject into an electrical signal, and generates an ultrasonic image by using the electrical signal.

The ultrasonic diagnostic apparatus including the ultrasonic probe, when used to perform the above-described processes, is useful for various medical purposes. For example, the ultrasonic diagnostic apparatus may be used for detecting foreign matter in living organisms, measuring a degree of injury, observing a tumor, and observing a fetus.

Extensive research has been conducted to improve the yield of the ultrasonic probes.

Provided are an ultrasonic probe to which a low temperature process is applicable, and a method of manufacturing the same.

Additional aspects will be set forth in part in the description which follows and, in part, will be apparent from the description, or may be learned by practice of the presented embodiments.

According to an aspect of an embodiment, an ultrasonic probe includes: a first unit configured to generate an ultrasonic wave from a first electrical signal or generate a second electrical signal from an echo signal of the ultrasonic wave; a second unit configured to provide the first electrical signal to the first unit or receive the second electrical signal from the first unit; and a third unit configured to electrically connect the first unit to the second unit, the third unit including a plurality of conductive bumps spaced apart from one another and a non-conductive paste or film that surrounds the plurality of conductive bumps.

The plurality of conductive bumps may be chemically bonded to metal materials included in the first unit and the second unit.

The chemical bond may be a metallic bond.

The plurality of conductive bumps may include a metal alloy having a liquefaction temperature of about 160°C or less.

The plurality of conductive bumps may include at least one selected from tin (Sn), bismuth (Bi), phosphorus (In), lead (Pb), silver (Ag), and gallium (Ga).

The plurality of conductive bumps may each have a diameter of about 120 µm or less.

At least one of the plurality of conductive bumps may have at least one selected from a ball shape and a pillar shape.

The first unit may include a plurality of first conductive pads that respectively contact the plurality of conductive bumps.

The first unit may further include an acoustic amplification layer that has a first surface contacting the plurality of first conductive pads.

The first unit may further include a piezoelectric layer arranged on a second surface of the acoustic amplification layer, wherein the second surface is opposite to the first surface.

The piezoelectric layer may include a plurality of piezoelectric elements that are two-dimensionally arranged.

The first unit may further include a piezoelectric layer that contacts the plurality of first conductive pads.

The second unit may include a plurality of second conductive pads that respectively contact the plurality of conductive bumps.

The second unit may further include a chip module substrate that has a first surface contacting the plurality of second conductive pads.

The second unit may further include an acoustic absorption layer that contacts a second surface of the chip module substrate , wherein the second surface is opposite to the first surface.

According to an aspect of another embodiment, a method of manufacturing an ultrasonic probe includes: preparing a first unit configured to generate an ultrasonic wave from a first electrical signal or generate a second electrical signal from an echo signal of the ultrasonic wave; preparing a second unit configured to provide the first electrical signal to the first unit or receive the second electrical signal from the first unit; forming a plurality of conductive bumps spaced apart from one another on the second unit; forming a non-conductive paste on the second unit to fill a space between the plurality of conductive bumps; and bonding the first unit to the second unit by using the plurality of conductive bumps and the non-conductivity paste.

The plurality of conductive bumps may be chemically bonded to metal materials included in the first unit and the second unit.

The chemical bond may be a metallic bond.

The plurality of conductive bumps may include a metal alloy having a liquefaction temperature of about 160°C or less.

The plurality of conductive bumps may include at least one selected from tin (Sn), bismuth (Bi), phosphorus (In), lead (Pb), silver (Ag), and gallium (Ga).

The plurality of conductive bumps may be formed by electroplating.

The plurality of conductive bumps may each have a diameter of about 120 µm or less.

The first unit may be bonded to the second unit at a temperature higher than a liquefaction temperature of the plurality of conductive bumps.

The first unit may be bonded to the second unit under atmospheric pressure.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram of an ultrasonic diagnostic apparatus according to an embodiment;
FIG. 2 is a block diagram of an ultrasonic probe illustrated in FIG. 1;
FIG. 3 is a diagram for describing a physical configuration of the ultrasonic probe illustrated in FIG. 2;
FIG. 4 is a diagram for describing an array of piezoelectric elements in a piezoelectric layer, according to an embodiment;
FIGS. 5 to 9 are diagrams for describing a method of manufacturing an ultrasonic probe, according to an embodiment;
FIG. 10 is a diagram for describing a physical configuration of an ultrasonic probe according to another embodiment; and
FIG. 11 is a diagram for describing a structure of a matching layer, according to another embodiment.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects. Expressions such as "at least one of" when preceding a list of elements, modify the entire list of elements and do not modify the individual elements of the list.

Hereinafter, embodiments will be described in detail with reference to the accompanying drawings. Like reference numerals in the drawings denote like elements, and descriptions thereof will not be repeated.

The term "subject" used herein may include a human, an animal, a part of the human, or a part of an animal. For example, the subject may include a blood vessel, or organs such as a liver, a heart, a womb, a brain, a breast, and an abdomen. In addition, the term "user" used herein may be a medical expert such as a doctor, a nurse, a medical technologist, or a medical image expert, or an engineer who repairs a medical apparatus, but the user is not limited thereto.

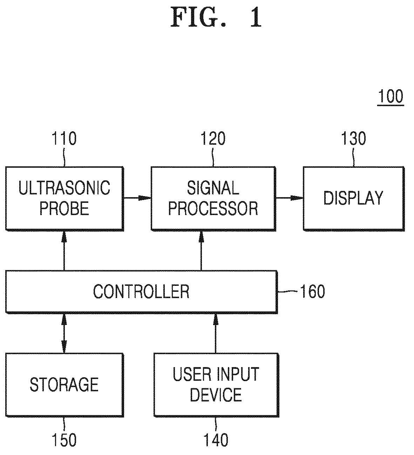

FIG. 1 is a block diagram of an ultrasonic diagnostic apparatus 100 according to an embodiment. Referring to FIG. 1, the ultrasonic diagnostic apparatus 100 may include an ultrasonic probe 110 configured to transmit and receive an ultrasonic wave, a signal processor 120 configured to process a signal applied by the ultrasonic probe 110 to generate an image, a display 130 configured to display the image, a user input device 140 configured to receive a user command, a storage 150 configured to store a variety of information, and a controller 160 configured to control an overall operation of the ultrasonic diagnostic apparatus 100.

The ultrasonic probe 110 may transmit an ultrasonic wave to a subject and receive an echo signal of the ultrasonic wave reflected from the subject. Detailed descriptions of the ultrasonic probe 110 will be provided below.

The signal processor 120 may generate an ultrasonic image by processing ultrasonic data generated by the ultrasonic probe 110. The ultrasonic image may be at least one selected from a brightness mode (B mode) image in which an amplitude of an echo signal of an ultrasonic wave reflected from a subject is represented by brightness, a Doppler mode image in which an image of a moving subject is represented by a spectrum form by using Doppler effect, a motion mode (M mode) image which shows a motion of a subject at a certain position according to time, an elastic mode image in which a reaction difference between a case where compression is applied to a subject and a case where compression is not applied to the subject is represented by an image, and a color mode (C mode) image in which a speed of a moving subject is represented by a color by using Doppler effect. Since an ultrasonic image is generated by using an existing ultrasonic image generating method, detailed descriptions thereof will be omitted. Therefore, an ultrasonic image according to an embodiment may include any dimensional images, such as a one-dimensional (1D) image, a two-dimensional (2D) image, a three-dimensional (3D) image, and a four-dimensional (4D) image.

The display 130 may display information processed by the ultrasonic diagnostic apparatus 100. For example, the display 130 may display the ultrasonic image generated by the signal processor 120 and may display a graphical user interface (GUI) so as to request a user input.

The display 130 may include at least one selected from a liquid crystal display, a thin film transistor-liquid crystal display, an organic light-emitting diode, a flexible display, a 3D display, and an electrophoretic display. According to embodiments, the ultrasonic diagnostic apparatus 100 may include two or more displays 130.

The user input device 140 may allow a user to input data so as to control the ultrasonic diagnostic apparatus 100. The user input device 140 may include a key pad, a mouse, a touch panel, a track ball, or the like. The user input device 140 is not limited to the illustrated configuration, but may further include various input devices, such as a jog wheel and a jog switch.

The touch panel may detect both a real touch and a proximity touch. The real touch means that a pointer actually touches a screen, and the proximity touch means that the pointer approaches the screen while being separate from the screen within a certain distance. The pointer is a device used to touch or proximately touch a specific portion of the touch panel. Examples of the pointer may include a stylus pen and a body part such as a finger.

In addition, the touch panel may be implemented by a touch screen that forms a layer structure with the display 130 described above. The touch screen may be classified into a capacitive type touch screen, a resistive type touch screen, an infrared beam type touch screen, a surface acoustic wave type touch screen, an integral strain gauge type touch screen, and a piezo effect type touch screen. Since the touch screen functions as both the display 130 and the user input device 140, the touch screen has high availability.

Although not illustrated in FIG. 1, various sensors may be provided inside or near the touch screen so as to detect the touch. An example of the sensor that detects the touch on the touch panel may include a tactile sensor. The tactile sensor may detect a contact of a specific object at or beyond a sensitivity of a person. The tactile sensor may detect a variety of information, such as roughness of a contact surface, hardness of a contact object, a temperature of a contact point, or the like.

Another example of the sensor that detects the touch on the touch screen may include a proximity sensor. The proximity sensor may detect the presence or absence of an object approaching a certain detection surface or an object existing nearby, by using a force of an electro-magnetic field or infrared rays, without any mechanical contact. Examples of the proximity sensor may include a transmission-type photoelectric sensor, a direct reflection type photoelectric sensor, a mirror reflection type photoelectric sensor, a high-frequency oscillation type proximity sensor, a capacitive type proximity sensor, a magnetic type proximity sensor, and an infrared proximity sensor.

The storage 150 may store a variety of information that is processed by the ultrasonic diagnostic apparatus 100. For example, the storage 150 may store medical data related to diagnosis of the subject, such as images, and may store algorithms or programs that are executed by the ultrasonic diagnostic apparatus 100.

The storage 150 may include at least one selected from a flash memory type storage medium, a hard disk type storage medium, a multimedia card micro type storage medium, a card type memory (e.g., an SD card, an XD memory, etc.), a random access memory (RAM), a static random access memory (SRAM), a read-only memory (ROM), an electrically erasable programmable ROM (EEPROM), a programmable read-only memory (PROM), a magnetic memory, a magnetic disc, and an optical disc. In addition, the ultrasonic diagnostic apparatus 100 may operate a web storage or a cloud server that performs a storage function of the storage 150 on a web.

The controller 160 may control an overall operation of the ultrasonic diagnostic apparatus 100. That is, the controller 160 may control operations of the ultrasonic probe 110, the signal processor 120, the display 130, and the like, which are illustrated in FIG. 1. For example, the controller 160 may control the signal processor 120 to generate an image by using a user command input through the user input device 140 or programs stored in the storage 150. Also, the controller 160 may control the display 130 to display the image generated by the signal processor 120.

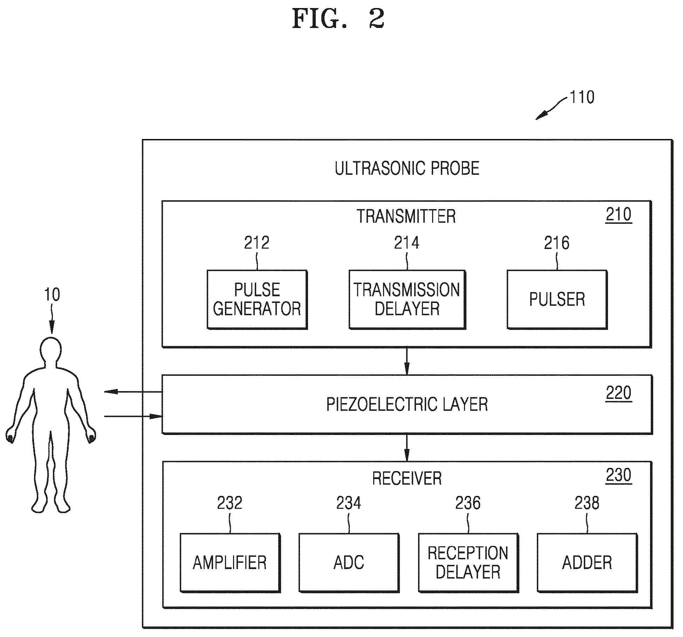

FIG. 2 is a block diagram of the ultrasonic probe 110 illustrated in FIG. 1. Referring to FIG. 2, the ultrasonic probe 110 is a device that generates ultrasonic data by transmitting an ultrasonic wave to a subject 10 and receiving an echo signal reflected from the subject 10. The ultrasonic probe 110 may include a transmitter 210, a piezoelectric layer 220, and a receiver 230.

The transmitter 210 may provide a driving signal to the piezoelectric layer 220. The transmitter 210 may include a pulse generator 212, a transmission delayer 214, and a pulser 216.

The pulse generator 212 may generate a rate pulse for generating a transmission ultrasonic wave based on a certain pulse repetition frequency (PRF). The transmission delayer 214 may apply a delay time to the rate pulse generated by the pulse generator 212, so as to determine transmission directionality. A plurality of rate pulses, to which the delay time is applied, correspond to a plurality of piezoelectric elements included in the piezoelectric layer 220, respectively. The pulser 216 may apply a driving signal (or a driving pulse) to the piezoelectric layer 220 at a timing corresponding to each of the plurality of rate pulses, to which the delay time is applied.

The piezoelectric layer 220 may transmit an ultrasonic wave to the subject 10 according to the driving signal provided by the transmitter 210, and receive an ultrasonic echo signal reflected from the subject 10. The piezoelectric layer 220 may include the plurality of piezoelectric elements configured to convert an electrical signal into an acoustic signal, and vice versa.

The receiver 230 may process a signal received from the piezoelectric layer 220 to generate ultrasonic data. The receiver 230 may include an amplifier 232, an analog-to-digital converter (ADC) 234, a reception delayer 236, and an adder 238.

The amplifier 232 may amplify the signal received from the piezoelectric layer 220, and the ADC 234 may perform an analog-to-digital conversion on the amplified signal to generate a digital signal. The reception delayer 236 may apply a delay time to the digital signal so as to determine reception directionality. The adder 238 may add signals processed by the reception delayer 236 to generate ultrasonic data. A reflection component from a direction determined based on the reception directionality may be emphasized by addition processing performed by the adder 238.

The transmitter 210 and the receiver 230 of the ultrasonic probe 110 may be formed in at least one chip on a single substrate 240. The substrate 240 may include silicon, ceramic, or a polymer-based material. The substrate 240 may include an acoustic absorption material that absorbs an ultrasonic wave. Each block inside the transmitter 210 and the receiver 230 may be formed in one chip, or two or more blocks may be formed in one chip. One chip may be formed corresponding to one piezoelectric element. Accordingly, a substrate including at least one selected from the transmitter 210 and the receiver 230 may be referred to as a chip module substrate 430. The chip module substrate 430 may mean a substrate including some of chips included in the ultrasonic probe 110 as well as a substrate including all chips included in the ultrasonic probe 110.

In addition to the transmitter 210 and the receiver 230, the ultrasonic probe 110 may further include a partial configuration of the signal processor 120, a partial configuration of the display 130, and a partial configuration of the user input device 140.

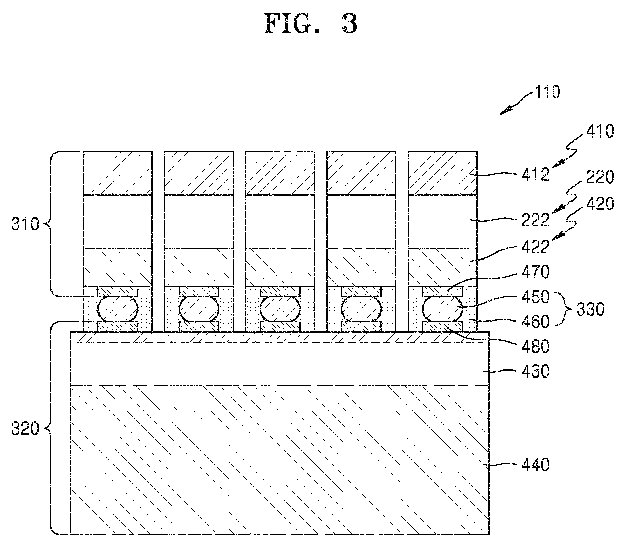

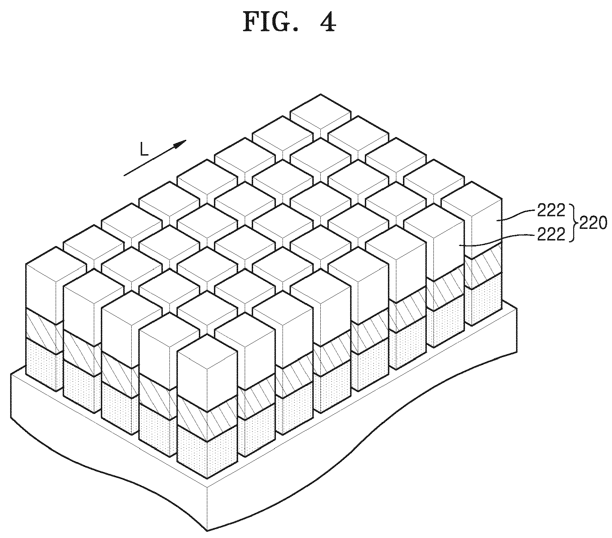

FIG. 3 is a diagram for describing a physical configuration of the ultrasonic probe 110 illustrated in FIG. 2. FIG. 4 is a diagram for describing an array of piezoelectric elements 222 included in the piezoelectric layer 220, according to an embodiment. As illustrated in FIG. 3, the ultrasonic probe 110 may include a first unit 310 configured to generate an ultrasonic wave from a first electrical signal or generate a second electrical signal from an echo signal of the ultrasonic wave, a second unit 320 configured to provide the first electrical signal to the first unit 310 or receive the second electrical signal from the first unit 310, and a third unit 330 configured to electrically connect the first unit 310 to the second unit 320. The echo signal of the ultrasonic wave is an ultrasonic wave reflected from a subject and may be referred to as an ultrasonic wave.

The first unit 310 may include the piezoelectric layer 220 configured to perform a conversion between an ultrasonic wave and an electrical signal, and a matching layer 410 configured to match an acoustic impedance of an ultrasonic wave generated by the piezoelectric layer 220 with an acoustic impedance of a subject. The first unit 310 may further include an acoustic amplification layer 420 configured to reflect an ultrasonic wave transmitted in a direction opposite to the subject.

The piezoelectric layer 220 may include the plurality of piezoelectric elements 222 configured to perform a conversion between an electrical signal and an ultrasonic wave. The plurality of piezoelectric elements 222 may be arranged spaced apart from one another. The piezoelectric elements 222 may include a material that causes piezoelectricity. The material that causes the piezoelectricity may include at least one selected from ZnO, AlN, PbZrTiO₃ (PZT), PbLaZrTiO₃ (PLZT), BaTiO₃ (BT), PbTiO₃ (PT), and PMN-PT.

As illustrated in FIG. 4, the piezoelectric elements 222 may be two-dimensionally arranged in a length direction L of the piezoelectric layer 220 and a direction perpendicular to the length direction L. This may be referred to as a two-dimensional (2D) piezoelectric layer 220. The piezoelectric layer 220 may have a linear array or a curved array. An array form may be variously set according to a designer's intension.

The piezoelectric layer 220 may appropriately delay an input time of each of signals input to the piezoelectric elements 222, and transmit the signal to the subject along an external scan line through which an ultrasonic wave is transmitted. In this manner, a three-dimensional (3D) image may be acquired by using a plurality of echo signals. As the number of piezoelectric elements 222 increases, a clearer ultrasonic image may be acquired. An array form may be variously set according to a designer's intension. According to another embodiment, the piezoelectric elements 222 may be one-dimensionally arranged.

The matching layer 410 may be disposed on a front surface of the piezoelectric layer 220. The matching layer 410 may approximate the acoustic impedance of the ultrasonic wave to the acoustic impedance of the subject by gradually changing the acoustic impedance of the ultrasonic wave generated by the piezoelectric layer 220. The matching layer 410 may have an acoustic impedance that is less than the acoustic impedance of the piezoelectric layer 220 and greater than the acoustic impedance of the subject. The front surface of the piezoelectric layer 220 may mean a surface of the piezoelectric layer 220 which is closest to the subject while an ultrasonic wave is emitted to the subject. A rear surface of the piezoelectric layer 220 may mean a surface opposite to the front surface.

The matching layer 410 may include a plurality of matching elements 412 which are respectively disposed on the plurality of piezoelectric elements 222. However, the matching layer 410 is not limited thereto. The plurality of piezoelectric elements 222 may be grouped, and a single matching element 412 may be provided on a resultant structure. The matching layer 410 may have a single-layered structure or a multi-layered structure.

The first unit 310 may further include an acoustic lens (not illustrated) configured to focus an ultrasonic wave. The acoustic lens may be disposed on the front surface of the piezoelectric layer 220 and may function to focus the ultrasonic wave generated by the piezoelectric layer 220. The acoustic lens may include a material such as a silicon rubber having an acoustic impedance close to the acoustic impedance of the subject. In addition, a central portion of the acoustic lens may be convex or flat. The acoustic lens may have various shapes according to a designer's design.

The acoustic amplification layer 420 may reflect an ultrasonic wave transmitted in a direction opposite to the subject. The acoustic amplification layer 420 may improve acoustic characteristics of an ultrasonic wave. The acoustic amplification layer 420 may include a material, such as tungsten carbide, which has high reflectivity with respect to an ultrasonic wave. The acoustic amplification layer 420 may support the piezoelectric layer 220 on the rear surface of the piezoelectric layer 220. Although not illustrated in FIG. 3, electrical lines may be disposed in the acoustic amplification layer 420 so as to electrically connect a chip module substrate 430 to the piezoelectric layer 220. The acoustic amplification layer 420 may include a plurality of acoustic amplification elements 422 which respectively correspond to the plurality of piezoelectric elements 222 of the piezoelectric layer 220.

The second unit 320 may include the chip module substrate 430 including at least one chip configured to process an electrical signal. The second unit 320 may further include an acoustic absorption layer 440 configured to absorb an ultrasonic wave.

As described above, the chip module substrate 430 may mean a substrate including at least one chip configured to process an electrical signal. For example, the chip module substrate 430 may include at least one chip configured to performs operations of the receiver 230 and the transmitter 210. The chip module substrate 430 may be an application specific integrated circuit (ASIC), but is not limited thereto.

The acoustic absorption layer 440 may absorb an ultrasonic wave that is transmitted in a direction opposite to the subject and is not directly used for examination or diagnosis. The acoustic absorption layer 440 may be disposed on a rear surface of the chip module substrate 430 and support the chip module substrate 430.

The acoustic absorption layer 440 is illustrated in FIG. 3 as being formed as an element separately from the chip module substrate 430 for convenience of description, but is not limited thereto. The chip module substrate 430 may include an acoustic absorption material. Accordingly, the chip module substrate 430 may also function as the acoustic absorption layer 440.

The ultrasonic probe 110 may further include the third unit 330 configured to electrically connect the first unit 310 to the second unit 320. The third unit 330 may include a plurality of conductive bumps 450 spaced apart from one other, and a non-conductive paste or film 460 that surrounds the plurality of conductive bumps 450 and fills a space between the first unit 310 and the second unit 320.

The first unit 310 may further include a plurality of first conductive pads 470 that respectively contact the plurality of conductive bumps 450. The second unit 320 may further include a plurality of second conductive pads 480 that respectively contact the plurality of conductive bumps 450. That is, the first conductive pads 470 may respectively contact upper ends of the conductive bumps 450. The second conductive pads 480 may respectively contact lower ends of the conductive bumps 450. The first conductive pads 470 and the second conductive pads 480 may include a metal material. For example, the first conductive pads 470 and the second conductive pads 480 may include at least one selected from gold (Au), nickel (Ni), copper (Cu), titanium (Ti), tantalum (Ta), aluminum (Al), and titanium tungsten (TiW).

The upper end of each of the plurality of conductive bumps 450 may be electrically connected to the first unit 310, for example, the piezoelectric layer 220. That is, the conductive bumps 450 may be respectively connected to the piezoelectric elements 222 through the first conductive pads 470 and the electrical lines (not illustrated) disposed in the acoustic amplification elements 422. The lower end of each of the plurality of conductive bumps 450 may be electrically connected to the second unit 320, for example, the chip module substrate 430, through each of the second conductive pads 480.

The conductive bumps 450 may include a metal alloy having a liquefaction temperature of about 160°C or less. For example, the conductive bumps 450 may include a metal alloy having a liquefaction temperature of about 160°C or less among metal alloys including metals such as tin (Sn), bismuth (Bi), phosphorus (In), lead (Pb), silver (Ag), and gallium (Ga). For example, the conductive bumps 450 may be liquefied at a temperature higher than the liquefaction temperature thereof and be chemically bonded to other metals. The chemical bond may be a metallic bond.

When the first unit 310 is bonded to the second unit 320 by using the conductive bumps 450 having a liquefaction temperature of about 200°C or more, the first unit 310 and the second unit 320 may be heated to a temperature of about 200°C or more. In this case, the piezoelectric layer 220 of the first unit 310 may lose piezoelectricity and may not recover the piezoelectricity even when an electrical stimulation is applied thereto. Therefore, when the conductive bumps 450 having a high liquefaction temperature are used, the yield of the ultrasonic probe 110 may be reduced.

In order to prevent the piezoelectric layer 220 from losing electrical characteristics, the first unit 310 may be bonded to the second unit 320 by applying a pressure at a low temperature. In this case, since the conductive bumps 450, the first conductive pads 470, and the second conductive pads 480 are solids, it is necessary to apply a high pressure. Due to the application of pressure, the conductive bumps 450 and the second conductive pads 480 may damage the chip module substrate 430. A physical binding force between the conductive bumps 450 and the first and second conductive pads 470 and 480 may be weakened by a vibration of the piezoelectric layer 220, causing a malfunction of the ultrasonic probe 110.

Since the ultrasonic probe 110 according to the present embodiment uses the conductive bumps 450 having a liquefaction temperature of about 160°C or less, the conductive bumps 450 may be chemically bonded to the first conductive pads 470 and the second conductive pads 480 in a liquefied state. The chemical bond may be a metallic bond. When the first unit 310, the second unit 320, and the conductive bumps 450 are heated to beyond the liquefaction temperature of the conductive bumps 450, the conductive bumps 450 may be chemically bonded to the first conductive pads 470 and the second conductive pads 480 in a liquefied state. A binding force of the chemical bond may be greater than a binding force of a physical bond. In addition, although the piezoelectric layer 220 is heated to about 160°C and thus loses piezoelectricity, the piezoelectric layer 220 may recover the piezoelectricity through an electrical stimulation. Therefore, it may be possible to improve the yield of the ultrasonic probe 110.

The conductive bumps 450 may have at least one selected from a ball shape and a pillar shape. The conductive bumps 450 may each have a diameter of about 120 µm or less. Since the conductive bumps 450 are formed by electroplating, the size of the conductive bumps 450 may be reduced. Due to the reduction in the size of the conductive bumps 450, it may be possible to improve the availability of the chip module substrate 430 and miniaturize the ultrasonic probe 110.

The non-conductive paste or film 460 may be disposed between the first unit 310 and the second unit 320 to surround the plurality of conductive bumps 450 and fill the space between the first unit 310 and the second unit 320. The non-conductive paste or film 460 may attach the first unit 310 to the second unit 320. An adhesive force of the non-conductive paste or film 460 may be less than an adhesive force of the conductive bumps 450.

The non-conductive paste or film 460 may include a phenol epoxy resin and a hardener, but is not limited thereto. The non-conductive paste or film 460 may include a thermosetting epoxy resin, such as acid anhydride, amine, or imidazole. The non-conductive paste or film 460 may have a thickness of about 20 µm to about 200 µm.

When the non-conductive paste is injected between the first unit 310 and the second unit 320, the non-conductive paste may be controlled to fill the space between the conductive bumps 450 and not to flow to the outside, because a viscosity of the non-conductive paste is greater than a viscosity of a liquid. Therefore, it may be possible to prevent the non-conductive paste from contaminating other elements of the ultrasonic probe 110. Moisture contained in the non-conductive paste is vaporized over time and the non-conductive paste becomes a non-conductive film. Therefore, reference numeral "460" may denote the non-conductive paste or the non-conductive film.

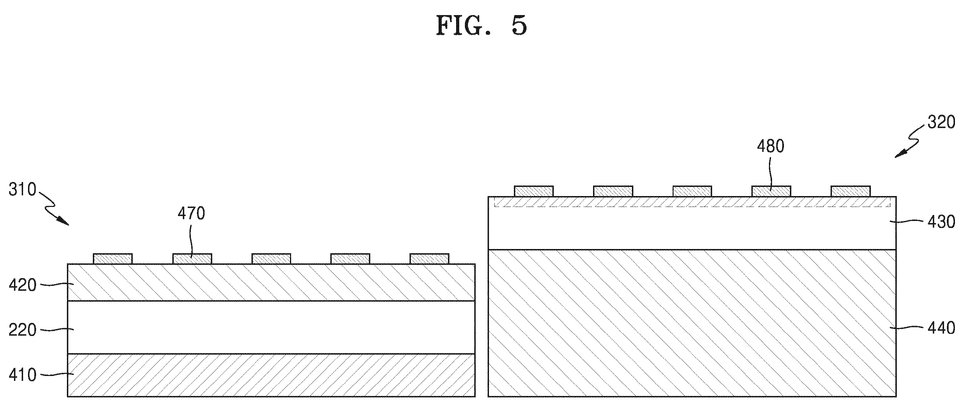

FIGS. 5 to 9 are diagrams for describing a method of manufacturing an ultrasonic probe 110, according to an embodiment.

As illustrated in FIG. 5, a first unit 310 and a second unit 320 may be prepared. The first unit 310 may generate an ultrasonic wave from a first electrical signal or generate a second electrical signal from an echo signal of the ultrasonic wave, and the second unit 320 may provide the first electrical signal to the first unit 310 or receive the second electrical signal from the first unit 310.

The first unit 310 may include a piezoelectric layer 220 configured to perform a conversion between an ultrasonic wave and an electrical signal, an acoustic amplification layer 420 disposed on a first surface of the piezoelectric layer 220 to reflect an ultrasonic wave, and a matching layer 410 disposed on a second surface of the piezoelectric layer 220 to match an acoustic impedance of an ultrasonic wave with an acoustic impedance of a subject. The first surface may be opposite to the second surface. The piezoelectric layer 220, the acoustic amplification layer 420, and the matching layer 410 of the first unit 310 may be stacked by using a mounting jig. A tolerance of the mounting jig may be within about 10 µm.

The second unit 320 may include a chip module substrate 430 including at least one chip configured to process an electrical signal, and an acoustic absorption layer 440 configured to absorb an ultrasonic wave or an echo signal of the ultrasonic wave.

When the first unit 310 is prepared, a plurality of first conductive pads 470 spaced apart from one another may be further formed on the acoustic amplification layer 420 of the first unit 310. Similarly, when the second unit 320 is prepared, a plurality of second conductive pads 480 spaced apart from one another may be further formed on the chip module substrate 430 of the second unit 320. The first conductive pads 470 and the second conductive pads 480 may include a metal material.

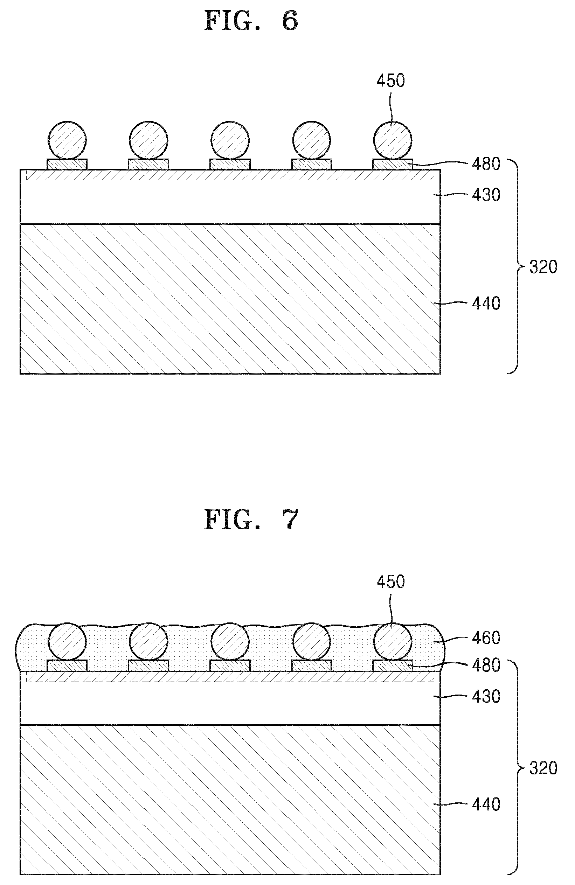

As illustrated in FIG. 6, a plurality of conductive bumps 450 may be formed on the second unit 320. The conductive bumps 450 may be respectively formed on the second conductive pads 480. The conductive bumps 450 having a solder ball shape may be formed on the second conductive pads 480. The conductive bumps 450 may be formed on the second conductive pads 480 by electroplating. Since the conductive bumps 450 are formed by electroplating, the conductive bumps 450 may be formed to have a small size. For example, the conductive bumps 450 may have a diameter of about 120 µm or less. When the size of the conductive bumps 450 is reduced, it may be possible to widen an available space of the chip module substrate 430 and miniaturize the ultrasonic probe 110. In addition, as illustrated in FIG. 7, a non-conductive paste 460 may be formed on the second unit 320 to fill a space between the plurality of conductive bumps 450. The non-conductive paste 460 may be formed by an underfill process. A viscosity of the non-conductive paste 460 is greater than a viscosity of an adhesive such as a resin. Therefore, a flow control of the non-conductive paste 460 may be facilitated.

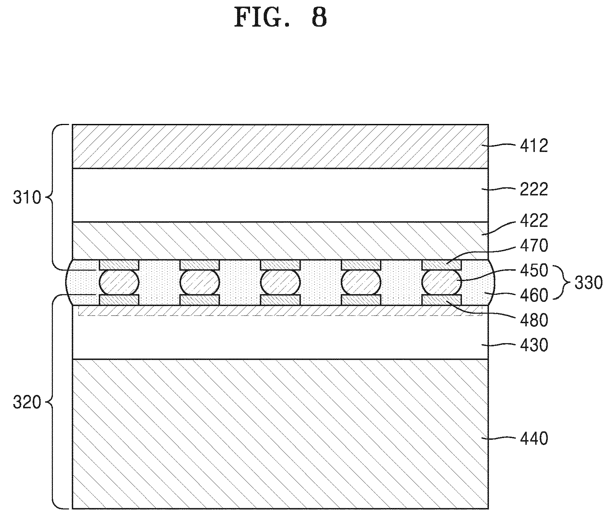

As illustrated in FIG. 8, the first unit 310 may be bonded to the second unit 320 by using the conductive bumps 450. The first unit 310 may be bonded to the second unit 320 by flip-chip bonding. For example, the first unit 310 may be bonded to the second unit 320 by placing a mounting jig, on which the first unit 310 is stacked, on the second unit 320. The first unit 310 and the second unit 320 may be aligned so that the first conductive pads 470 contact the conductive bumps 450. The first unit 310, the second unit 320, and the conductive bumps 450 may be heated to a temperature higher than a melting point of the conductive bumps 450. As a result, the conductive bumps 450 may become a liquefied state and be chemically bonded to the first conductive pads 470. The chemical bond may be a metallic bond.

When the first conductive pads 470 of the first unit 310 and the second conductive pads 480 of the second unit 320 contact the conductive bumps 450, a temperature is higher than the melting point of the conductive bumps 450. Thus, the conductive bumps 450 may become a liquefied state and be chemically bonded to the first conductive pads 470 and the second conductive pads 480.

When the first unit 310 is bonded to the second unit 320, a pressure may be atmospheric pressure. A pressure applied to the first unit 310 may be the sum of the atmospheric pressure and the weight of the first unit 310. Since the conductive bumps 450 are bonded to the first conductive pads 470 and the second conductive pads 480 in the liquefied state, it is unnecessary to apply a high pressure to the first unit 310. A slight pressure may be applied to the first unit 310 so as to uniformly bond the first unit 310 to the second unit 320.

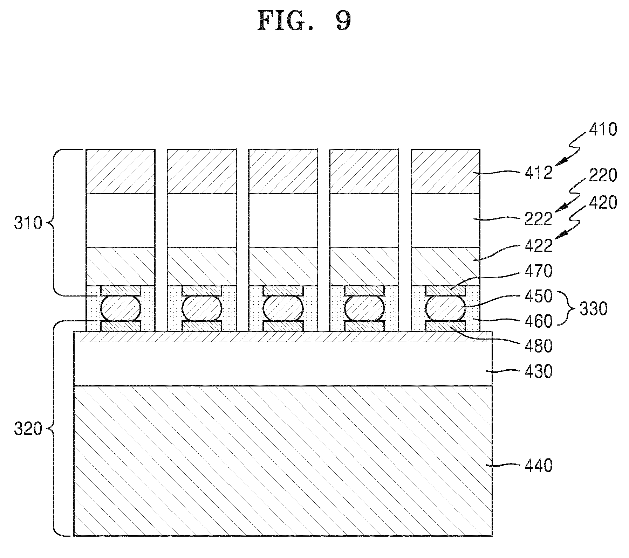

As illustrated in FIG. 9, the first unit 310 may be cut to form piezoelectric elements 222, matching elements 412, and acoustic amplification elements 422. Since the process is performed at a low temperature and a low pressure, the productivity of the ultrasonic probe 110 according to the present embodiment may be increased.

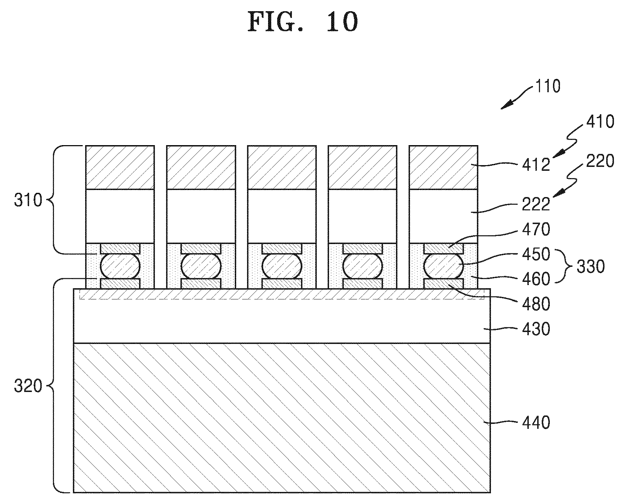

FIG. 10 is a diagram for describing a physical configuration of an ultrasonic probe 110 according to another embodiment. The ultrasonic probe 110 of FIG. 10 differs from the ultrasonic probe 110 of FIG. 3 in that a first unit 310 does not include an acoustic amplification layer 420. That is, the first unit 310 illustrated in FIG. 10 may include a piezoelectric layer 220, a matching layer 410, an acoustic lens, and a plurality of first conductive pads 470.

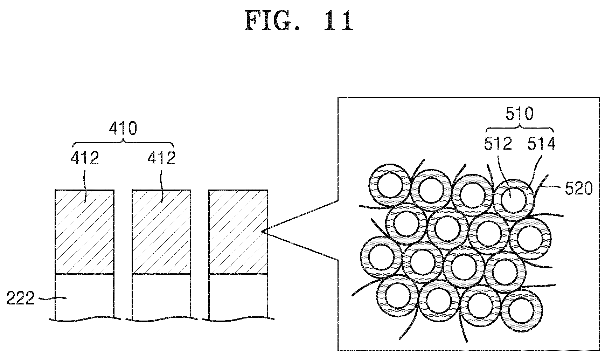

The matching layer 410 of the ultrasonic probe 110 according to the present embodiment may match acoustic impedances and may have electrical conductivity. FIG. 11 is a diagram for describing a structure of a matching layer 410 according to another embodiment. Referring to FIG. 11, the matching layer 410 may have a particle-in-binder (PIB) structure in which a plurality of conductive particles 510 are mixed with a binder 520.

Each of the plurality of conductive particles 510 may contact at least one neighboring conductive particle 510. Therefore, the matching layer 410 may have conductivity as a whole. The conductive particles 510 in the matching layer 410 may have the same size, and at least two conductive particles 510 may have different sizes. The conductive particles 510 in the matching layer 410 may be the same material, and at least two conductive particles 510 may be different materials. Electrical conductivity and an acoustic impedance of the matching layer 410 may be determined according to a kind of a material, a size of the conductive particles 510, a content of the conductive particles 510, a material of the binder 520, and a content of the binder 520.

Each of the plurality of conductive particles 510 may include a core 512 and a shield 514. A material of the core 512 may be different from a material of the shield 514. The shield 514 may surround a surface of the core 512. Electrical conductivity of the core 512 may be different from electrical conductivity of the shield 514.

The electrical conductivity of the core 512 may be less than the electrical conductivity of the shield 514. The core 512 may include at least one selected from a non-conductive material, a semiconductor material, and a conductive material. Even when the core 512 includes the conductive material, the electrical conductivity of the core 512 may be less than the electrical conductivity of the shield 514. For example, the core 512 may include a conductive material, such as copper (Cu), aluminum (Al), nickel (Ni), tungsten (W), beryllium (Be), phosphorus (In), iron (Fe), lead (Pb), titanium (Ti), or tin (Sn). Alternatively, the core 512 may include a non-conductive material, such as glass, or may include a semiconductor material, such as silicon (Si), germanium (Ge), or boron (B). The core 512 may include a metal oxide, such as alumina (Al₂O₃), tin oxide (SnO₂), iron hydroxide (Fe₂O₃), or zinc oxide (ZnO). The metal oxide may belong to a conductive material, a semiconductor material, and a non-conductive material.

The electrical conductivity of the shield 514 may be greater than the electrical conductivity of the core 512. For example, the shield 514 may include at least one selected from silver (Ag), gold (Au), and platinum (Pt), which are metals having high electrical conductivity.

The conductive particles 510 are illustrated in FIG. 11 as having a spherical shape, but this is merely an example. Besides the spherical shape, the conductive particles 510 may have one selected from a flake shape, a bar shape, a rod shape, a wire shape, a fiber shape, and a conical shape. The binder 520 may fill a space between the conductive particles 510 in the matching layer 410. The binder 520 may be a material that matches an acoustic impedance. For example, the binder 520 may include at least one selected from a polyvinyl butyral-based material, an acryl-based material, a polyester-based material, a phenoxy-based material, a polyvinyl formal-based material, a polyamide-based material, a polystyrene-based material, a polycarbonate-based material, a polyvinyl acetate-based material, a polyurethane-based material, an epoxy-based material, and any mixtures thereof.

When the matching layer 410 has electrical conductivity, the piezoelectric layer 220 may be electrically connected to the chip module substrate 430 through the matching layer 410. Therefore, the number of conductive bumps 450 may be reduced.

It should be understood that embodiments described herein should be considered in a descriptive sense only and not for purposes of limitation. Descriptions of features or aspects within each embodiment should typically be considered as available for other similar features or aspects in other embodiments.

While one or more embodiments have been described with reference to the figures, it will be understood by those of ordinary skill in the art that various changes in form and details may be made therein without departing from the spirit and scope as defined by the following claims.

## Claims

1. An ultrasonic probe comprising:
a first unit configured to generate an ultrasonic wave from a first electrical signal or generate a second electrical signal from an echo signal of the ultrasonic wave;
a second unit configured to provide the first electrical signal to the first unit or receive the second electrical signal from the first unit; and
a third unit configured to electrically connect the first unit to the second unit, the third unit comprising a plurality of conductive bumps spaced apart from one another and a non-conductive paste or film that surrounds the plurality of conductive bumps.

2. The ultrasonic probe of claim 1, wherein the plurality of conductive bumps are chemically bonded to metal materials included in the first unit and the second unit.

3. The ultrasonic probe of claim 2, wherein the chemical bond is a metallic bond.

4. The ultrasonic probe of any one of claims 1 to 3, wherein the plurality of conductive bumps comprise a metal alloy having a liquefaction temperature of about 160°C or less.

5. The ultrasonic probe of claim 4, wherein the plurality of conductive bumps comprise at least one selected from tin (Sn), bismuth (Bi), phosphorus (In), lead (Pb), silver (Ag), and gallium (Ga).

6. The ultrasonic probe of any one of claims 1 to 5, wherein the plurality of conductive bumps each have a diameter of about 120 µm or less.

7. The ultrasonic probe of any one of claims 1 to 6, wherein the first unit comprises a plurality of first conductive pads that respectively contact the plurality of conductive bumps.

8. The ultrasonic probe of claim 7, wherein the first unit further comprises an acoustic amplification layer that has a first surface contacting the plurality of first conductive pads.

9. The ultrasonic probe of claim 7 or 8, wherein the first unit further comprises a piezoelectric layer that contacts the plurality of first conductive pads.

10. A method of manufacturing an ultrasonic probe, the method comprising: preparing a first unit configured to generate an ultrasonic wave from a first electrical signal or generate a second electrical signal from an echo signal of the ultrasonic wave;
preparing a second unit configured to provide the first electrical signal to the first unit or receive the second electrical signal from the first unit;
forming a plurality of conductive bumps spaced apart from one another on the second unit;
forming a non-conductive paste on the second unit to fill a space between the plurality of conductive bumps; and
bonding the first unit to the second unit by using the plurality of conductive bumps and the non-conductivity paste.

11. The method of claim 10, wherein the plurality of conductive bumps are chemically bonded to metal materials included in the first unit and the second unit.

12. The method of claim 10 or 11, wherein the plurality of conductive bumps comprise a metal alloy having a liquefaction temperature of about 160°C or less.

13. The method of any one of claims 10 to 12, wherein the plurality of conductive bumps are formed by electroplating.

14. The method of any one of claims 10 to 13, wherein the first unit is bonded to the second unit at a temperature higher than a liquefaction temperature of the plurality of conductive bumps.

15. The method of any one of claims 10 to 14, wherein the first unit is bonded to the second unit under atmospheric pressure.
